# EUROPEAN PATENT SPECIFICATION

(11) **EP 1 429 696 B1**
(45) Date of publication and mention of the grant of the patent: **28.03.2007**
(21) Application number: 02779226.6
(22) Date of filing: 23.09.2002
(51) Int. Cl.: A61F 5/448, A61F 5/441, A61F 5/443

(54) **AN OSTOMY APPLIANCE**
OSTOMIEVORRICHTUNG
ACCESSOIRE POUR OSTOMIE

(30) Priority: 24.09.2001 DK 200101389
(43) Date of publication of application: 23.06.2004
(73) Proprietor: COLOPLAST A/S, 3050 Humlebaek (DK)
(72) Inventor: CIOK, Danuta, 2990 Nivaa (DK); HANSEN, Michael, 3250 Gilleleje (DK)
(74) Representative: Nilausen, Kim
(86) International application number: PCT/DK2002/000623
(87) International publication number: WO 2003/026541

(56) References cited:
- WO-A-98/17212
- WO-A-98/55057
- GB-A- 2 290 974
- US-A- 3 983 298
- US-A- 6 071 268

## Description

### BACKGROUND OF THE INVENTION

### 1. Field of the Invention

The present invention relates to an ostomy appliance comprising a body side member comprising an adhesive wafer for securing the appliance to a user's skin, said wafer having a hole for receiving a stoma, and a separately exchangeable receiving bag secured to the body side ostomy member for receiving fluids or excretions emerging from an abdominal stoma and a sealing member for sealing between a stoma and an ostomy body side member.

In connection with surgery for a number of diseases in the gastro-intestinal or urinary tract a consequence is, in many cases, that the colon, the ileum or the ureter has been exposed surgically and the patient is left with an abdominal stoma, or, in nephrostomy or ureterostomy, the ureter or a catheter is exposed in the back or the chest region or abdominal region, and the effluents or waste products of the body, which are conveyed through these organs, are discharged through the artificial orifice or opening and are collected in a collection bag, which is usually adhered to the skin by means of an adhesive wafer or plate having an inlet opening for accommodating the stoma/ureter/catheter. Also in connection with a fistula, the patient will have to rely on an appliance to collect the bodily material emerging from such opening.

Ostomy appliances are well known. Such appliances may be two-piece or one-piece appliances. In both types of appliances, an adhesive barrier member (or base plate) is attached to the wearer's abdomen/back/chest. In case of a one-piece appliance, a receiving member or bag is attached to the base plate. In case of a two-piece appliance, the adhesive barrier member forms part of a body side member and a receiving member or bag is attached releasably to the body side ostomy member for receiving exudates from the stoma.

When using one-piece appliances, the whole appliance, including the adhesive skin barrier securing the appliance to the skin is normally removed and replaced by a fresh appliance. When using two-piece appliances, the body side ostomy member is left in place up to several days, and only the receiving member or bag attached to the body side member is replaced. The attachment means for attaching an ostomy receiving bag may e.g. be a system se comprising matching coupling rings or matching flanges and adhesive surfaces engaging with and sealing against a flange area of the body side member.

### 2. Description of the Related Art

Published International Patent Application No. WO 98/17212 discloses an ostomy appliance comprising a body side member comprising an adhesive wafer or pad for securing the appliance to the user's skin, said wafer or pad having a hole for receiving a stoma, and an optionally separately exchangeable receiving member or bag secured to the body side ostomy member for receiving secretions from the ostomy said ostomy appliance further comprising a separate sealing member disposed in the hole of the wafer or pad surrounding the stoma, said sealing member being of a hypo-allergenic adhesive and being in the form of a mouldable mass or a ring.

Published International Patent Application No. WO 00/49981 discloses an ostomy appliance comprising a body side member, an optionally separately exchangeable receiving member or bag secured to the body side member, said body side member comprising a ring-shaped body having an adhesive wafer having a hole for accommodating a stoma and further a separate sealing member is in the form of a disc which, when in use, is placed in the hole for sealing against the stoma, wherein the disc has an outer diameter smaller than the diameter of the hole of the wafer or pad of the ostomy body side member and a flange member stretching from its outer rim, and wherein the flange has outer dimensions greater than the diameter of the hole of the body side member for coupling to the body side member.

Published International Patent Application No. WO 98/53771 also discloses an ostomy appliance comprising a body side member comprising an adhesive wafer or pad for securing the appliance to the user's skin, said wafer or pad having a hole for receiving a stoma, and an optionally separately exchangeable receiving member or bag secured to the body side ostomy member for receiving secretions from the ostomy said ostomy appliance further comprising a sealing member disposed in the hole of the wafer or pad surrounding the stoma wherein the sealing member disposed in the hole of the wafer or pad surrounding the stoma, said sealing member having a hole for accommodating the stoma and said sealing member having balanced plastic and elastic properties allowing an adaptation of the hole of the ostomy appliance to a stoma by a temporary enlarging the hole by everting or rolling up the inner rim of the hole for accommodating the stoma.

Published International Patent Application No. WO 98/53772 discloses an ostomy appliance comprising a body side member comprising an adhesive wafer or pad for securing the appliance to the user's skin, said wafer or pad having a hole for receiving a stoma, and an separately exchangeable receiving member or bag secured to the body side ostomy member for receiving secretions from the ostomy said ostomy appliance further comprising a separate sealing member disposed in the hole of the wafer or pad surrounding the stoma wherein the separately exchangeable receiving member or bag is secured releasably to the body side ostomy member by a mechanical fastening means.

Although the above references disclose the use of a separate sealing member there is still need of a separate sealing member that may be used together with conventional two-piece ostomy appliances in order to allow an extension of the service time of the body side member for use with an increased number of receiving bags and thus reduce the frequency of stressing the skin around the stoma due to exchange of the body side member by protecting the surface of the adhesive wafer from soiling by and attack from the aggressive visceral contents and facilitating the cleaning of the body side member. Furthermore, there is a need of an improved protecting sealing member improving the safety and quality of life of ostomates having an active social life without having to carry both fresh collecting bags and fresh sealing members while being out of the daily whereabouts.

### SUMMARY OF THE INVENTION

The present invention relates to an ostomy appliance comprising a body side member comprising an adhesive wafer for securing the appliance to the user's skin, said wafer having a hole for receiving a stoma, wherein the body side member comprises first substantially annular coupling means for releasable attachment of a separately exchangeable receiving bag to the body side ostomy member for receiving secretions from the stoma, said receiving bag comprising matching second substantially annular coupling means, wherein the body side member comprises a separate sealing member for sealing against the stoma, wherein the separate sealing member is in the form of a disc having a centre hole for accommodating the stoma and wherein at least the surface of the disc facing the skin of the user comprises a mass of a skin-friendly adhesive.

Furthermore, the invention relates to a sealing member for sealing between a stoma and an ostomy body side member comprising an adhesive wafer for securing the appliance to the user's skin, said wafer having a hole for receiving a stoma, wherein the body side member comprises first substantially annular coupling means for releasable attachment of a separately exchangeable receiving bag to the body side ostomy member for receiving secretions from the stoma which sealing member is in the form of a disc having a centre hole for accommodating the stoma and wherein at least the surface of the disc facing the skin of the user comprises a mass of a skin-friendly adhesive.

### Brief Description of the Drawings

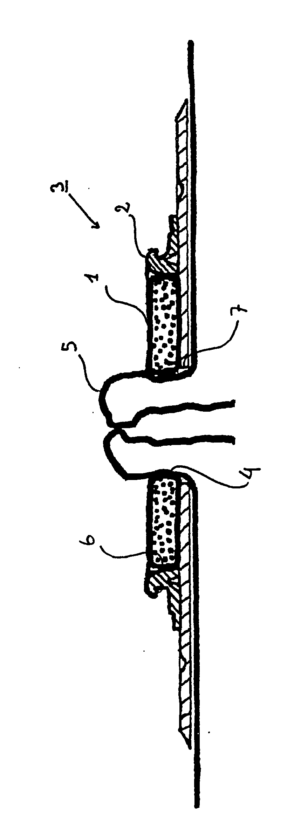

The invention is disclosed more in detail with reference to the drawings in which the figure shows a sectional view of an embodiment of the invention in which a sealing disc is placed within the limits of a coupling ring of an ostomy body side member being situated on the user.

### Detailed Description of the Present Invention

The invention relates to an ostomy appliance comprising a body side member comprising an adhesive wafer for securing the appliance to the user's skin, said wafer having a hole for receiving a stoma, wherein the body side member comprises first substantially annular coupling means for releasable attachment of a separately exchangeable receiving bag to the body side ostomy member for receiving secretions from the stoma, said receiving bag comprising matching second substantially annular coupling means, wherein the body side member comprises a separate sealing member for sealing against the stoma, wherein the separate sealing member is in the form of a disc having a centre hole for accommodating the stoma and wherein at least the surface of the disc facing the skin of the user comprises a mass of a skin-friendly adhesive, wherein the disc has a maximum outer diameter corresponding to the inner diameter of the first annular coupling means and wherein the centre hole of the disc has a diameter smaller than the diameter of the stoma-receiving hole of the body side member ensuring that the disc covers all of the surface of the adhesive wafer facing away from the user located between the first annular coupling means and the stoma, said disc being made from a material that may be detached, rinsed and reapplied.

It has been found that an ostomy appliance of the invention fulfils the above needs and also provides further advantages as appears from the below. The sealing member of the invention protects the body side member against contact with and attack by the aggressive visceral contents and has a service time at least corresponding to and even exceeding the service time of the body side member. Thus, it better protects the body side member from soiling and also eases the cleaning thereof using water when substituting the receiving bag. This also renders it possible to extend the service time of the sealing member to cover at least the service time of the body side member and several changes of collecting bag. Thus, when changing bag while being out of the daily whereabouts, the ostomate may reuse the sealing member and thus does not have to carry both fresh collecting bags and fresh sealing members.

As the outer diameter of the disc corresponds to the inner diameter of the coupling means, the adaptation of the hole of the body side member to the stoma less critical as the disc covers all of the surface of the body side member inside the coupling means and thus protects the surface of the body side member from contact with visceral contents reducing the need of cleaning when substituting the collection bag with a fresh. This also enables the option of using body side members having a large hole generally and to avoid the individual adaptation of the size of the hole in most cases. Furthermore, only a relatively few number of sizes of sealing members of the invention, corresponding to the number of sizes of coupling rings, are needed, which sealing members furthermore only have to be adapted with respect to the size of the centre hole in order to provide a safe sealing which simplifies the use of the sealing members.

It is preferred that the diameter of the centre hole of the sealing member corresponds to the diameter of the stoma which ensures a complete protection of the body side member as well as of the skin surrounding the stoma.

In one embodiment of the invention the coupling means is in the form of matching flanges and adhesive surfaces.

In a preferred embodiment of the invention the annular coupling means is in the form of matching coupling rings.

In accordance with a further preferred embodiment of the invention, the thickness of the sealing member is of the same size as the distance between the surface of the adhesive wafer facing away from the user and the top of the coupling ring or the flange as the sealing member then covers and protects the full surface of the first coupling means facing the stoma and thus reduces the risk of soiling of parts of the body side member and consequently also reduces the necessary cleaning .

It is preferred that the whole disc is made from an adhesive being resistant to attack from visceral contents as it is supposed to be in service for extended periods of time.

In order to facilitate cleaning of the body side member when substituting receiving bags it is preferred that the skin-friendly adhesive is suitable for repeated adhering to and removal from the skin. Thus, the sealing member may be removed from the body side member and cleaned and repositioned and used for further protection of the body side member. It is especially preferred that the skin-friendly adhesive is made from a permanently tacky silicone adhesive as such material may be deaning using water and does not bind materials or odour emerging from a stoma. Thus a clean environment is easy to maintain.

In accordance with a preferred embodiment the surface of the disc facing away from the user is covered with a non-tacky layer in order to reduce the risk of adherence of materials emerging from a stoma or the opposite bag wall if it is brought in contact with the disc by accident.

One practical solution is obtained when the surface facing away from the user is covered with a non-tacky polymer film, e.g. a film of a polyolefin such as polyethylene or polypropylene or a silicone polymer.

The disc is preferably made form a silicone material as silicone materials are generally skin-friendly and very stable when contacted by aggressive exudates from a stoma and furthermore, silicones may be cleaned by rinsing with water without having to rely on the use of detergents which further facilitates repeated cleaning and use of a sealing member. Furthermore, silicones are often transparent enabling an inspection of the area around the stoma without having to remove the sealing disc which reduces the stress of the skin connected with removal and replacing the sealing disc. Still further, when producing sealing members from silicones, it is not necessary to rely on protecting or containment sheet materials.

A preferred material for the non-tacky layer is a silicone rubber being relatively inert for aggressive fluids and being washable using water. Suitable materials are platinum catalysed vinyl endblocked polydiorganosiloxanes of the kind described in US patent no. 3,983,298, which have high tack, good adhesive strength. The properties and tack of these polysiloxanes may be adjusted according to wish by routine experiments by the skilled in the art for preparation of more tacky materials for adhering to the skin and less tacky or non-tacky layers for the surface facing away from the user. A preferred material is vinyl endblocked crosslinked polydimethylsiloxanes. Furthermore, it is preferred that the surface of the disc facing away from the user is hydrophobic as this facilitates the cleansing thereof.

The sealing member used in accordance with the invention may be elastic enabling a temporary enlargement of the hole for receiving the stoma whereafter the disc reverts to substantially its original shape as long as the elastic force is not so strong that there is a risk of constricting the stoma. Its is also considered an embodiment of the invention that the sealing member is plastically mouldable allowing a final adaptation of the shape thereof to fit more snugly to the stoma.

It is also contemplated that the sealing member used in accordance with the invention is somewhat plastic allowing for a minor displacement of material to allow a full engagement with the surface of the stoma.

It is preferred that the material is slightly conformable and adapts itself to the actual surface of the stoma. This active adaptation is more efficient when the sealing member is of a thickness corresponding to the height of a coupling ring.

The choice of materials having the above-referenced properties may made by the skilled in the art based on routine experiments.

An ostomy body side member according to the invention may be produced from standard materials normally used for preparation of disposable ostomy and wound and incontinence devices. Thus, the carrier sheet may be any suitable thermoplastic material and the adhesive wafer may be made from a medical grade barrier adhesives known in the such as the formulation being disclosed, for example in US Patents Nos. 4,367,732, 5,051,259 or 5,714,225.

The coupling means may be any system known per se for attaching receiving bags to ostomy body side members and may suitably be matching coupling rings of the type disclosed in WO 93/18725 or WO 94/18919 or matching flanges for adhesive connection of the type disclosed in U.S. Patent No. 5,800,415.

In a further aspect the invention relates to a sealing member for sealing between a stoma and an ostomy body side member comprising an adhesive wafer for securing the appliance to the user's skin, said wafer having a hole for receiving a stoma, wherein the body side member comprises first substantially annular coupling means for releasable attachment of a separately exchangeable receiving bag to the body side ostomy member for receiving secretions from the stoma which sealing member is in the form of a disc having a hole for accommodating the stoma and wherein at least the surface of the disc facing the skin of the user comprises a mass of a skin-friendly adhesive wherein the disc has a maximum outer diameter corresponding to the inner diameter of the first annular coupling means and wherein the centre hole of the disc has a diameter smaller than the diameter of the stoma-receiving hole of the body side member with which it is intended to be used.

### Description of the Preferred Embodiments

The invention is now explained more in detail with reference to the drawings showing preferred embodiments of the invention.

Reference is made to the Figure of the drawings showing a sectional view of an embodiment of an ostomy body side member of the invention in which a sealing disc **1** is placed within the limits of a coupling ring **2** of the body side member **3** being situated on the user. As appears, the disc has an opening **4** for receiving a stoma **5** and fills out the space between the stoma **5** the coupling ring **2** for releasable attachment of a receiving bag. Thus, the disc has an outer diameter corresponding to the inner diameter of the coupling ring so that the disc and the coupling ring are in sealing contacting in the interface **6** and the diameter of the centre hole or opening **4** is smaller than the diameter of the stoma-receiving hole **7** of the body side member.

As also appears, the sealing disc **1** protects all of the surface of the body side member facing away from the user from direct contact with material exiting the stoma and thus from soiling or attack from such material.

## Claims

1. An ostomy appliance comprising a body side member (3) comprising an adhesive wafer for securing the appliance to the user's skin, said wafer having a hole (7) for receiving a stoma (5), wherein the body side member comprises first substantially annular coupling means (2) for releasable attachment of a separately exchangeable receiving bag to the body side ostomy member for receiving secretions from the stoma, said receiving bag comprising matching second substantially annular coupling means, wherein the body side member comprises a separate sealing member for sealing against the stoma, wherein the separate sealing member is in the form of a disc (1) having a centre hole (4) for accommodating the stoma (5) and wherein at least the surface of the disc facing the skin of the user comprises a mass of a skin-friendly adhesive, wherein the disc has a maximum outer diameter corresponding to the inner diameter of the first annular coupling means and wherein the centre hole (4) of the disc (1) has a diameter smaller than the diameter of the stoma-receiving hole (7) of the body side member ensuring that the disc (1) covers all of the surface of the adhesive wafer facing away from the user located between the first annular coupling means and the stoma, **characterised in that** the disc is made from a material that may be detached, rinsed with water without detergents and reapplied.

2. An ostomy appliance as claimed in claim 1 wherein the diameter of the centre hole (4) of the sealing member corresponds to the diameter of the stoma (5).

3. An ostomy appliance as claimed in claim 1 or 2 wherein the annular coupling means (2) is in the form of matching flanges and adhesive surfaces.

4. An ostomy appliance as claimed in claim 1 or 2 wherein the annular coupling means (2) is in the form of matching coupling rings.

5. An ostomy appliance as claimed in claim 4 wherein the thickness of the sealing member (1) is of the same size as the distance between the surface of the adhesive wafer facing away from the user and the top of the coupling ring (2).

6. An ostomy appliance as claimed in any of claims 1-5 wherein the seating member (1) is made from an adhesive being resistant to attack form visceral contents.

7. An ostomy appliance as claimed in claim 6 wherein the sealing member (1) is made from a skin-friendly adhesive being suitable for repeated adhering to and removal from the skin.

8. An ostomy appliance as claimed in claim 7 wherein the skin-friendly adhesive is made from a permanently tacky silicone adhesive.

9. An ostomy appliance as claimed in any of claims 6-8 wherein the surface of the disc (1) facing away from the user is covered with a non-tacky layer.

10. An ostomy appliance as claimed in claim 9 wherein the surface facing away from the user is covered with a non-tacky polymer film.

11. An ostomy appliance as claimed in claim 9 wherein the non-tacky layer is a silicone rubber.

12. A separately exchangeable sealing member (1) in combination with an ostomy body side member (3) comprising an adhesive wafer for securing the appliance to the user's skin, said wafer having a hole (7) for receiving a stoma (5), wherein the body side member (1) comprises substantially annular coupling means (2) for releasable attachment of a separately exchangeable receiving bag to the body side ostomy member for receiving secretions from the stoma, wherein the sealing member is in the form of a disc (1) having a centre hole (4) for accommodating the stoma (5) and wherein at least the surface of the disc facing the skin of the user comprises a mass of a skin-friendly adhesive, wherein the disc has a maximum outer diameter corresponding to the inner diameter of the first annular coupling means (2) and wherein the centre hole (4) of the disc (1) has a diameter smaller than the diameter of the stoma-receiving hole (7) of the body side member (3) with which the sealing member is intended to be used,
**characterised in that** the disc is made from a material that may be detached, rinsed with water without detergents and reapplied.

## Patentansprüche

1. Stomavorrichtung, die ein körperseitiges Element (3) aufweist, das ein Klebeplättchen zur Befestigung der Vorrichtung auf der Haut des Anwenders aufweist, das ein Loch (7) zur Aufnahme eines Stomas (5) besitzt, wobei das körperseitige Element eine erste, im Wesentlichen ringförmige Verbindungseinrichtung (2) zur lösbaren Befestigung eines separat austauschbaren Aufnahmebeutels am körperseitigen Element zur Aufnahme von Ausscheidungen aus dem Stoma aufweist und der Aufnahmebeutel eine passende zweite, im Wesentliche ringförmige Verbindungseinrichtung besitzt und wobei das körperseitige Element ein separates Abdichtelement zur Abdichtung gegen das Stoma aufweist, wobei das separate Abdichtelement die Form einer Scheibe (1) mit einem zentralen Loch (4) zur Aufnahme des Stomas (5) besitzt und zumindest die Oberfläche der Scheibe, die zur Haut des Anwenders hin liegt, eine Masse aus einem hautfreundlichen Kleber aufweist, die Scheibe einen maximalen Außendurchmesser besitzt, der dem Innendurchmesser der ersten ringförmigen Verbindungseinrichtung entspricht, und das zentrale Loch (4) der Scheibe (1) einen Durchmesser besitzt, der kleiner ist als der Durchmesser des das Stoma aufnehmenden Lochs (7) des körperseitigen Elements, was sicherstellt, dass die Scheibe (1) die gesamte vom Anwender abgekehrte Oberfläche des Klebeplättchens, die sich zwischen der ersten ringförmigen Verbindungseinrichtung und dem Stoma befindet, bedeckt,
**dadurch gekennzeichnet, dass**
die Scheibe aus einem Material besteht, das abgenommen, mit Wasser ohne Reinigungsmittel abgespült und wieder angebracht werden kann.

2. Stomavorrichtung nach Anspruch 1, bei welcher der Durchmesser des zentralen Lochs (4) des Abdichtelements dem Durchmesser des Stomas (5) entspricht.

3. Stomavorrichtung nach Anspruch 1 oder 2, bei der die ringförmige Verbindungseinrichtung (2) in Form von passenden Flanschen und Kleberoberflächen vorliegt.

4. Stomavorrichtung nach Anspruch 1 oder 2, bei der die ringförmige Verbindungseinrichtung (2) in Form von passenden Verbindungsringen vorliegt.

5. Stomavorrichtung nach Anspruch 4, bei der die Dicke des Abdichtelements (1) so groß ist wie der Abstand zwischen der vom Anwender abgekehrten Oberfläche des Klebeplättchens und der Oberseite des Verbindungsrings (2).

6. Stomavorrichtung nach einem der Ansprüche 1 bis 5, bei der das Abdichtelement (1) aus einem Kleber besteht, der gegen den Angriff von Visceralinhalt beständig ist.

7. Stomavorrichtung nach Anspruch 6, bei der das Abdichtelement (1) aus einem hautfreundlichen Kleber besteht, der sich zum wiederholten Aufkleben auf die Haut und Lösen von der Haut eignet.

8. Stomavorrichtung nach Anspruch 7, bei welcher der hautfreundliche Kleber aus einem Siliconkleber mit permanentem Klebevermögen besteht.

9. Stomavorrichtung nach einem der Ansprüche 6 bis 8, bei der die vom Anwender abgekehrte Oberfläche der Scheibe (1) mit einer nichtklebenden Schicht abdeckt ist.

10. Stomavorrichtung nach Anspruch 9, bei der die vom Anwender abgekehrte Oberfläche mit einem nichtklebenden Polymerfilm abgedeckt ist.

11. Stomavorrichtung nach Anspruch 9, bei der die nichtklebende Schicht aus einem Siliconkautschuk besteht.

12. Separat austauschbares Abdichtelement (1) in Kombination mit einem körperseitigen Element (3) einer Stomavorrichtung, das ein Klebeplättchen zur Befestigung der Vorrichtung auf der Haut des Anwenders aufweist, das ein Loch (7) zur Aufnahme eines Stomas (5) besitzt, wobei das körperseitige Element (1) eine im Wesentliche ringförmige Verbindungseinrichtung (2) zur lösbaren Befestigung eines separat austauschbaren Aufnahmebeutels am körperseitigen Element zur Aufnahme von Ausscheidungen aus dem Stoma aufweist und wobei das Abdichtelement die Form einer Scheibe (1) mit einem zentralen Loch (4) zur Aufnahme des Stomas (5) besitzt und zumindest die Oberfläche der Scheibe, die zur Haut des Anwenders hin liegt, eine Masse aus einem hautfreundlichen Kleber aufweist, wobei die Scheibe einen maximalen Außendurchmesser besitzt, der dem Innendurchmesser der ersten ringförmigen Verbindungseinrichtung (2) entspricht, und das zentrale Loch (4) der Scheibe (1) einen Durchmesser besitzt, der kleiner ist als der Durchmesser des das Stoma aufnehmenden Lochs (7) des körperseitigen Elements (3), mit dem das Abdichtelement verwendet werden soll,
**dadurch gekennzeichnet, dass** die Scheibe aus einem Material besteht, das abgenommen, mit Wasser ohne Reinigungsmittel abgespült und wieder angebracht werden kann.

## Revendications

1. Accessoire de stomie comprenant un élément côté corps (3) comportant une rondelle adhésive pour la fixation de l'accessoire sur la peau d'un patient, ladite rondelle présentant un orifice (7) pour la réception d'une stomie (5), l'élément côté corps comportant un premier moyen de raccordement (2) de forme sensiblement annulaire pour la fixation amovible d'une poche collectrice séparément renouvelable sur l'élément de stomie côté corps, destinée à recevoir les sécrétions de la stomie, la poche collectrice comprenant un deuxième moyen de raccordement accouplé de forme sensiblement annulaire, dans lequel l'élément côté corps comprend un élément de joint séparé pour l'obturation contre la stomie, dans lequel l'élément de joint séparé a la forme d'un disque (1) avec un orifice central (4) pour le logement de la stomie (5), et dans lequel au moins la surface du disque se présentant face à la peau du patient est pourvue d'une couche d'adhésif hypoallergénique, et dans lequel le disque présente un diamètre extérieur maximal correspondant au diamètre intérieur du premier moyen de raccordement annulaire, dans lequel l'orifice central (4) du disque (1) présente un diamètre inférieur au diamètre de l'orifice (7) de réception de la stomie de l'élément côté corps, assurant que le disque (1) recouvre toute la face de la rondelle adhésive tournant le dos à la peau du patient entre le premier moyen de raccordement annulaire et la stomie, **caractérisé en ce que** le disque est réalisé dans un matériau lui permettant d'être détaché, rincé à l'eau sans détergents avant d'être repositionné.

2. Accessoire de stomie comme revendiqué dans la revendication 1, dans lequel le diamètre de l'orifice central (4) de l'élément de joint correspond au diamètre de la stomie (5).

3. Accessoire de stomie comme revendiqué dans la revendication 1 ou 2, dans lequel le moyen de raccordement annulaire (2) se présente sous forme de brides et de surfaces adhésives ajustées.

4. Accessoire de stomie comme revendiqué dans la revendication 1 ou 2, dans lequel le moyen de raccordement annulaire (2) se présente sous forme de bagues de raccordement ajustées.

5. Accessoire de stomie comme revendiqué dans la revendication 4, dans lequel l'épaisseur de l'élément de joint (1) est égale à la distance entre la face de la rondelle adhésive tournant le dos à la peau du patient et le sommet de la bague de raccordement (2).

6. Accessoire de stomie comme revendiqué dans l'une quelconque des revendications 1 à 5, dans lequel l'élément de joint (1) est réalisé dans un matériau adhésif résistant aux agressions des sécrétions viscérales.

7. Accessoire de stomie comme revendiqué dans la revendication 6, dans lequel l'élément de joint (1) est réalisé dans un matériau adhésif hypoallergénique approprié à des applications et à des retraits répétés sur la peau.

8. Accessoire de stomie comme revendiqué dans la revendication 7, dans lequel l'adhésif hypoallergénique est un adhésif siliconé à adhérence permanente.

9. Accessoire de stomie comme revendiqué dans l'une quelconque des revendications 6 à 8, dans lequel la face du disque (1) tournant le dos à la peau du patient est revêtue d'une couche non adhérente.

10. Accessoire de stomie comme revendiqué dans la revendication 9, dans lequel la face tournant le dos à la peau du patient est revêtue d'un film polymère non adhérent.

11. Accessoire de stomie comme revendiqué dans la revendication 9, dans lequel la couche non adhérente est un caoutchouc silicone.

12. Elément de joint (1) séparément renouvelable associé à un élément de stomie côté corps (3) comportant une rondelle adhésive pour la fixation de l'accessoire sur la peau d'un patient, ladite rondelle présentant un orifice (7) pour la réception d'une stomie (5), dans lequel l'élément côté corps (1) comprend un moyen de raccordement (2) de forme sensiblement annulaire pour la fixation amovible d'une poche collectrice séparément renouvelable sur l'élément de stomie côté corps, destinée à recevoir les sécrétions de la stomie, dans lequel l'élément de joint a la forme d'un disque (1) muni d'un orifice central (4) pour le logement de la stomie (5), et dans lequel au moins la face du disque tournée vers la peau du patient est pourvue d'une couche d'adhésif hypoallergénique, dans lequel le disque présente un diamètre extérieur maximal correspondant au diamètre intérieur du premier moyen de raccordement annulaire (2), et dans lequel l'orifice central (4) du disque (1) a un diamètre inférieur au diamètre de l'orifice (7) de réception de la stomie de l'élément côté corps (3) avec lequel l'élément de joint est prévu pour être utilisé, **caractérisé en ce que** le disque est réalisé dans un matériau lui permettant d'être détaché, rincé à l'eau sans détergents avant d'être repositionné.
